# EUROPEAN PATENT SPECIFICATION

(11) **EP 1 948 237 B1**
(45) Date of publication and mention of the grant of the patent: **04.05.2011**
(21) Application number: 05784461.5
(22) Date of filing: 10.08.2005
(51) Int. Cl.: A61K 45/06, A61K 31/715, A61K 31/716, A61K 31/736

(54) **USE OF ß-1,3 (4)-ENDOGLUCANOHYDROLASE, ß-1,3 (4) GLUCAN, DIATOMACEOUS EARTH, MINERAL CLAY AND GLUCOMANNAN TO AUGMENT IMMUNE FUNCTION**
VERWENDUNG VON - ß-1,3 (4)-ENDOGLUCANOHYDROLASE, ß-1,3 (4) GLUCAN, KIESELGUR, MINERALTON UND GLUCOMANNAN ZUR STEIGERUNG DER IMMUNFUNKTION
EMPLOI DE ß-1,3(4)-ENDOGLUCANOHYDROLASE, DE ß-1,3(4)-GLUCANE, DE TERRE DIATOMÉE, D'ARGILE MINÉRALE ET DE GLUCOMANNANE POUR AMÉLIORER LA FONCTION IMMUNITAIRE

(43) Date of publication of application: 30.07.2008
(73) Proprietor: Omnigen Research, LLC, Corvallis OR 97330 (US)
(72) Inventor: Omnigen Research, LLC, Corvallis OR 97330 (US)
(74) Representative: Prins, Hendrik Willem
(86) International application number: PCT/US2005/028529
(87) International publication number: WO 2007/021262

(56) References cited:
- WO-A-95/30022
- WO-A-97/02356
- US-A1- 2002 048 573
- DATABASE WPI Section Ch, Week 200002 Derwent Publications Ltd., London, GB; Class B06, AN 2000-021619 XP002359382 & RU 2 115 421 C1 (DEVICHENSKII V M) 20 July 1998 (1998-07-20)
- DATABASE WPI Section Ch, Week 199824 Derwent Publications Ltd., London, GB; Class B06, AN 1998-269963 XP002359383 & RU 2 093 162 C1 (AS SIBE BIOCHEM INST) 20 October 1997 (1997-10-20)

## Description

### References Cited [Referenced by]

### U.S. Patent Documents

| | | | |
|---|---|---|---|
| 4,857,512 | Aug 15, 1989 | Wagner *et al.* | 514/54 |
| 5,183,667 | Feb. 2, 1993 | Koch, H. | 424/474 |
| 5,519,009 | Oct 1, 1993 | Donzis, B. | 514/54 |
| 6,395,311 | May 28, 2002 | Jia, Q. | 424/744 |
| 6,541,678 | Apr. 1, 2003 | Klein, B. | 602/41 |
| 6,573,245 | Jun 3, 2003 | Marciani, J. | 514/25 |
| 6,660,722 | Dec. 9,2003 | Yvin, J.C. | 514/54 |

### Other References:

Adib Conquy, M., C. Fitting. Immunological status of cardiac arrest and resuscitated patients.http://www.pasteur.fr/recherche/RAR/RAR2002/Cytoinf-en.html, 2002. AOAC. 2002. Official Methods of Analysis. 17th Edition. AOAC International Press.
Burton, J.L., R.J. Erskine. Immunity and mastitis. Some new ideas for an old disease. Vet. Clin. Food Anim. 19:1-45, 2003.
Invivogen. http://www.invivogen.com/genedescription/TLR01.htm, 2004.
Travis, J. Biologists reveal the proteins that first see dangerous microbes. Week of Sept. 8, 2001 Vol. 160, No. 10
Werling D., J.C. Hope, C.J. Howard, T.W. Jungi. Differential production of cytokines, reactive oxygen and nitrogen by bovine macrophages and dendritic cells stimulated with Toll-like receptor agonists. Immunology111:41-52, 2004.
White S.N., K.H. Taylor, C.A. Abbey, C.A. Gill, J.E.Womack. Haplotype variation in bovine Toll-like receptor 4 and computational prediction of a positively selected ligand-binding domain. Proc. Natl. Acad. Sci. USA. 100:10364-10369, 2003
Weber, P.S.D., S.A. Madsen, G. W Smith, J.J. Ireland, J.L. Burton. Pre-translational regulation of neutrophil L-selectin in glucocorticoid-challenged cattle. Vet. Immunol. Immunopath. 83:213-240,2001.

### FIELD OF THE INVENTION

This disclosure relates to methods and compositions for the augmentation of immune function in mammalian and avian species.

### BACKGROUND OF THE INVENTION

The immune system consists of two general features. These are: 1) the innate immune system and 2) the adaptive (antibody-mediated) immune system. The innate system represents the first line of defense against an invading pathogen (whether bacterial or fungal) and provides the adaptive immune system with enough time (3-5 days) for it to build up antibodies which are used to "fight" pathogens. While the innate and adaptive systems are often described separately, they function in tandem; striving to sequester and neutralize a pathogen challenge.

**The innate immune system.** The innate immune system consists of several interesting components: Aspects include:
1. Physical and chemical barriers to pathogens provided by epithelium, gastric acid and digestive enzymes.
2. Cells which engulf and digest invading pathogens (e.g., neutrophils).
3. Receptors on the surface of these cells which recognize and bind to pathogens.
4. Signaling molecules (e.g., chemokines, cytokines) which communicate sites of infection and regulate expression of immune genes.

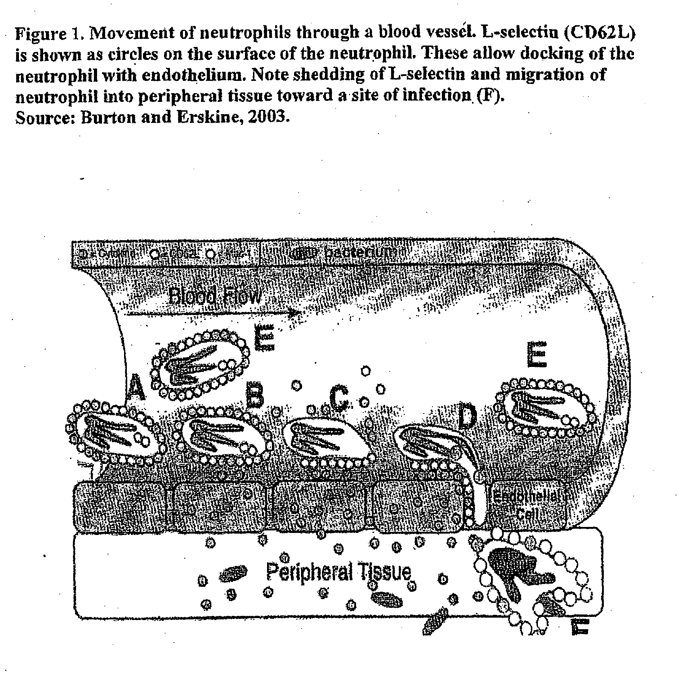

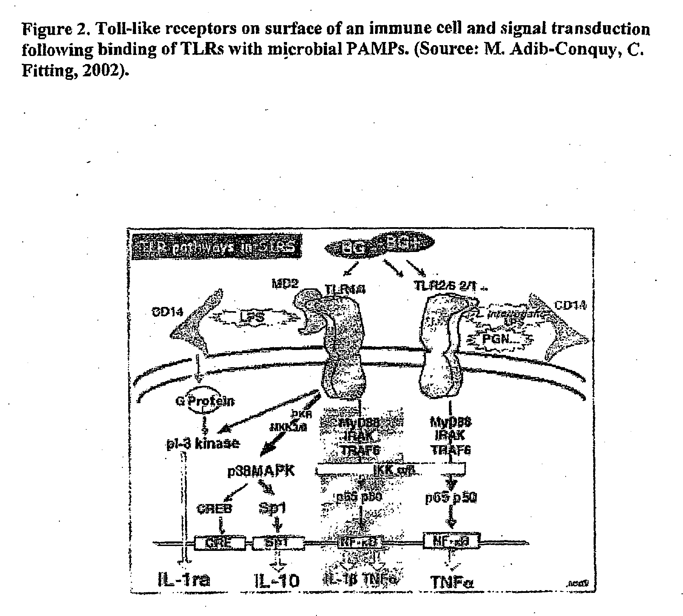

**Neutrophils.** Neutrophils are among the most important cells of the innate immune system. They are the first cell to arrive at a site of infection. In mammals, there are billions of neutrophils of which about one-half are freely circulating in the blood (Burton and Erskine, 2003). The remainder are held in reserve in bone marrow where they are formed. Neutrophils express an extracellular binding protein on their membranes termed "L-selectin" (also termed CD62L). The role of L-selectin is to interact weakly with the endothelial cell wall thereby allowing the neutrophil to "roll" along the wall of a blood vessel and to "monitor" the cell wall for the presence of signals which indicate a local infection (Figure 1). The presence of pathogens in peripheral tissues causes release of local chemicals which then signal a rolling neutrophil of an infection. In response to these signals, L-selectin is shed from the surface of the neutrophil (see Figure 1) and other more adhesive molecules are expressed on its surface. These molecules essentially "glue" the neutrophil within the blood vessel adjacent to the site of infection. The activated neutrophil then migrates through the endothelial cell wall toward the invading pathogen. Interleukin-1β is [produced by the neutrophil as a pro-inflammatory cytokine. This aids in mediating inflammation and in facilitating containment of invading pathogens. During neutrophil migration, chemical signals originating from the site of infection (such as TNF-α and interferon-y) activate the neutrophil to become a mature "killer cell". The mature neutrophil migrates toward the site of infection where it interacts with pathogen-associated microbial patterns (PAMPs) on the surface of pathogens via several types of receptors. These receptors are expressed on the surface of the neutrophil and include the following well-identified types (Figure 2):
**a**- CD18 and CD14
**b**- Toll-like receptors (TLRs)
c- C3b and C3bi (complement factors)
d- Fc

**Binding of neutrophils to pathogens via receptors.** Both CD14 and CD18 receptors bind with lipopolysaccharide (LPS), a common polysaccharide structure associated with membranes of gram-negative bacteria. In addition, neutrophils express toll-like receptors (TLRs) which recognize and bind to additional structures associated with pathogens. So far, ten different toll-like receptors have been identified in mammals (Figure 2 and Table 1). TLRs play a critical role in early innate immunity to invading pathogens by sensing microorganisms. These evolutionarily conserved receptors recognize highly conserved structural motifs only expressed by microbial pathogens, called pathogen-associated microbial patterns (PAMPs: Invivogen, 2004). Stimulation of TLRs by PAMPs initiates a signaling cascade that involves a number of proteins, such as MyD88 and IRAK (Figure 2). This signaling cascade leads to the activation of the transcription factor NF-kB which induces the secretion of cytokines that direct the adaptive (i.e., antibody-mediated) immune response. TLRs are predominantly expressed in tissues involved in immune function, such as spleen and peripheral blood leukocytes, as well as those exposed to the external environment such as lung and the gastrointestinal tract. Ten human and nine mouse TLRs have been characterized, seven of which have had their ligands identified. For examples, TLR2 is essential for the recognition of a variety of PAMPs, including bacterial lipoproteins, peptidoglycan, and lipotechoic acids. TLR3 is implicated in virus-derived double-stranded RNA. TLR4 is predominantly activated by lipopolysaccharide. TLR5 detects bacterial flagellin and TLR9 is required for response to unmethylated CpG DNA (Table 1). Recently, TLR7 and TLR8 were shown to recognize synthetic antiviral molecules. These receptors are essential elements in host defense against pathogens by activating the innate immunity (Invivogen, 2004).

*Bovine TLRs.* Relatively few studies on PAMPs have been completed with bovine cells. So far, bovine immune cells have been reported to contain TLR2 and TLR4 (Werling *et al*., 2004). Polymorphisms have been reported in bovine TLR4 which may determine susceptibility to bovine respiratory disease and Johne's disease (White *et al.,* 2003).

C3b and C3bi are components of the complement cascade whereas the Fc receptor binds to the "constant region" of antibodies. Hence, pathogens which are coated with complement factors or antibody (i.e., pathogens which are opsonized) are also recognized by activated neutrophils and are subsequently phagocytosed. In other words, activated neutrophils possess several means by which they recognize pathogens (Table 1).

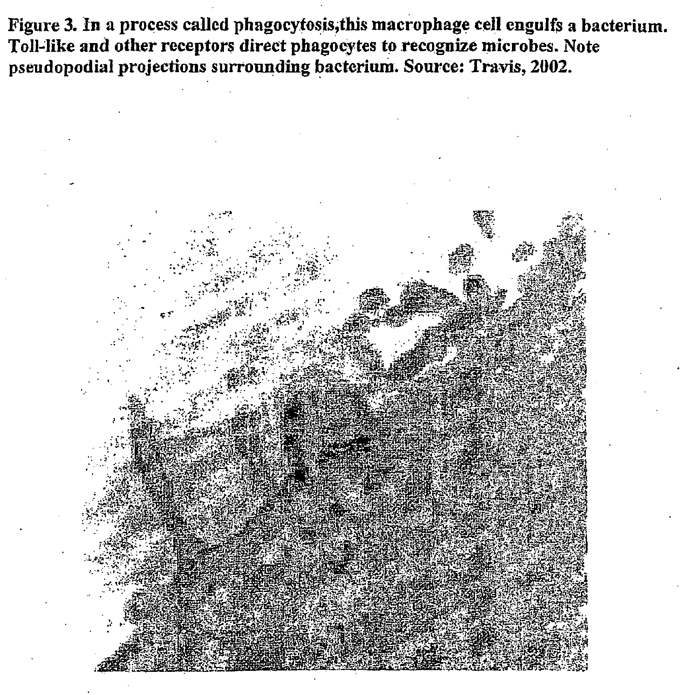

**Phagocytosis and killing.** The binding of neutrophils, (and other phagocytic cells) to cell-surface markers of pathogens via these receptors, then permits the phagocytic cell to engulf the invading pathogen and "kill" it (Figure 3). Presently, two mechanisms for "killing" are known. These include: 1) an oxidative burst, where the phagocyte expresses reactive oxygen species which destroy the phagocytosed pathogen, and 2) fusion of the engulfed pathogen with a lysosome-like structure to form a "phagosome". The phagosome is rich in digestive enzymes which mediate complete digestion of pathogens.

**Common infections.** Mammalian and avian species are continually challenged by pathogens in the gastrointestinal track and in the lung. These are important sites for resident neutrophils where minimize pathogen invasion. In addition, the mammary gland of mammals represents a site for pathogen challenge. In all infections, the innate immune system plays a key initial role in fighting-off the initial immune challenge. The innate system is essential to allow the adaptive (antibody-mediated) system to develop and mount a more-specific and directed immune response.

**Cooperation between the innate and acquired immune system in ruminants.** Antibodies which are specific to an invading pathogen leak into a site of infection to optimize clearance of a pathogen. Individuals with a high titer against a specific antigen are able to deliver these antibodies into the site of infection via a leaky endothelium (arising from an inflammatory response). Arrival of reactive antibodies (i.e., IgG₂) in the alveolus coats (opsonizes) the pathogen and, as noted previously, allows neutrophil recognition of pathogens via Fc receptors (Table 1) and phagocytosis.

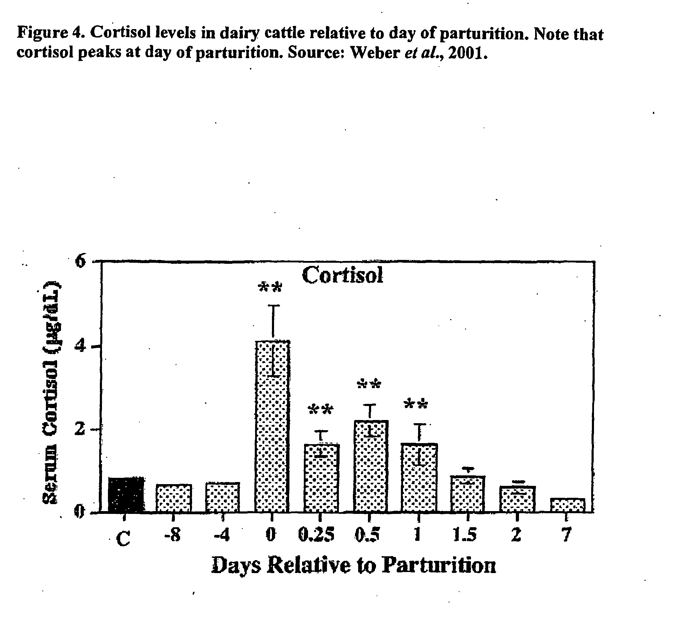

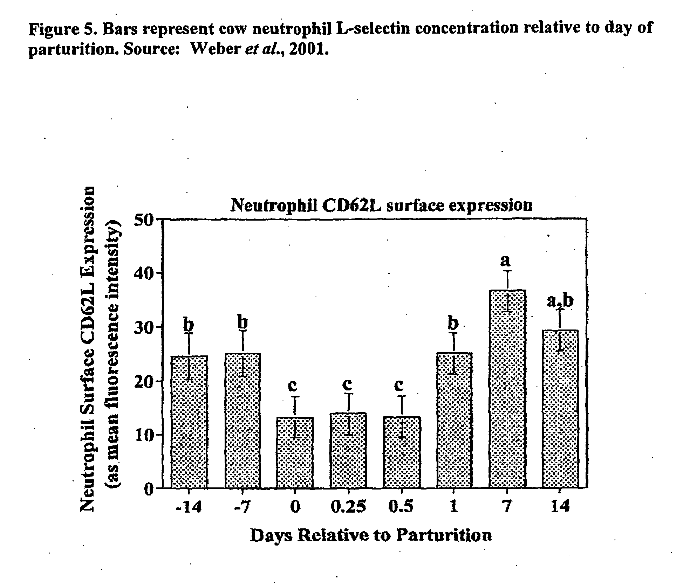

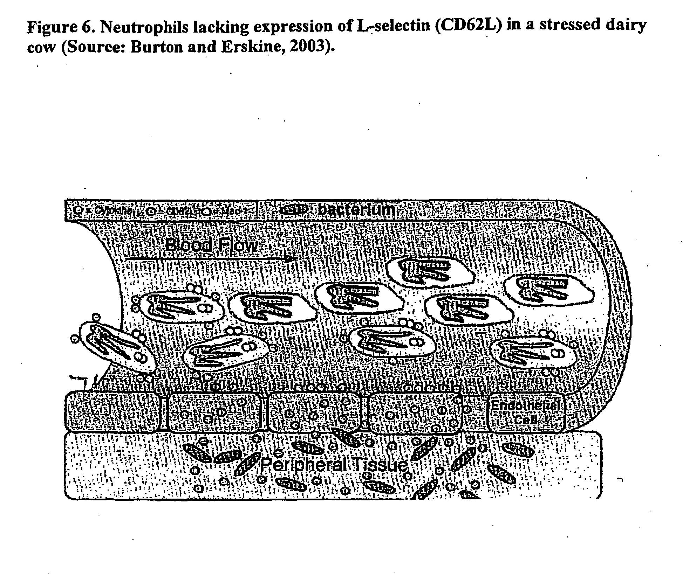

**Stress and immune function.** Stress reduces individuals' abilities to fight disease. The negative effects of stress on the immune system are mediate by the steroidal stress hormones (cortisol, hydrocortisone and corticosterone). Burton and co-workers at Michigan State University (Weber *et al.,* 2001) have identified the mechanism by which stress brings about a reduction in immune function. Specifically, they have documented that glucocorticoids (i.e., cortisol) "spike" near parturition (Figure 4) and reduce L-selectin expression in neutrophils (Figure 5). This compromises one important aspect of an individual's first line-of-defense against pathogen challenge. Specifically, a stressed, immunosuppressed individual has reduced ability to monitor endothelial cell lining for sites of infection and to attack and to sequester pathogens. This may result in an infection (Figure 6).

### SUMMARY OF THE INVENTION

The object of the present invention is to provide a novel and previously unknown method for augmentation of the immune system in mammalian and avian species. The invention may be applied to, but not limited to, mammalian and avian species and will reduce susceptibility of an individual to both fungal and bacterial diseases.

A further object of this invention is to provide a method for augmentation of immune function and to thereby minimize or obviate morbidities and mortalities caused by, but not limited to, pathogenic fungi and bacteria with a preparation comprising a combination of β-1,3 (4)-endoglucanohydrolase, β-glucan, diatomaceous earth, glucomannan, and mineral clay, such as aluminum silicate, montmorillonite clay, bentonite or zeolite.

Another object of the invention is to provide a composition comprising a combination of β-1,3 (4)-endoglucanohydrolase, β-glucan, diatomaceous earth, mineral clay, and glucomannan, which additively augments immune function and thereby, reduces potential of pathogenic fungi and bacteria to cause morbidities and mortalities in mammalian and avian species.

Additional objects, advantages and novel features of the invention will be set forth, in part, in the description that follows and will, in part, become apparent to those skilled in the art upon examination of the following or may be learned with the practice of the invention. To achieve the foregoing and other objects, and in accordance with the purposes of the present invention as described herein, a novel method is described for the augmentation of immune function of mammalian and avian species. In particular, this invention increases expression of neutrophil L-selectin and interleukin-1β and thereby minimizes or eliminates the colonization of the epithelial surfaces and underlying parenchymal tissues by pathogenic fungi and bacteria, reduces the populations of pathogenic organisms in blood and thereby minimizes or eliminates pathologies directly caused by and indirectly caused by this colonization. The invention comprises a mixture of β-1,3 (4)-endoglucanohydrolase, β-glucan, diatomaceous earth, mineral clay, and glucomannan. The diatomaceous earth is standard commercial grade available from a variety of sources. The β-1,3 (4)-endoglucanohydrolase is produced from submerged fermentation of a strain of *Trichoderma longibrachiatum.* The β-1,3 (4)glucan and glucomannan are derived from a commercial product and are an extraction from any of a number of yeast organisms. The mineral clay product is a standard commercial grade (examples include, but are not limited to, montmorillonite clay, bentonite and zeolite). Extractions and productions of diatomaceous earth, yeast cell wall extract and mineral clay are well known in the art and commercially-available.

The compositions which are provided by the invention can be fed to any mammalian or avian species including, but not limited to, bovine, equine, ovine, caprine and avian species. When admixed with the feed or food or fed as a supplement, the invention augments immune function thereby reducing colonization by pathogens. The invention also minimizes or eliminates invasion of the blood compartment by pathogenic fungi and bacteria. The invention thereby minimizes or eliminates the manifestations of the pathologies typically associated with epithelial and systemic fungal and bacterial infections. Administration of the product may be used as a prophylactic (i.e., to prevent colonization and growth of pathogenic fungal and bacterial species in mammalian or avian species), as an additive to feeds or foods infected with pathogenic fungi or bacteria or as a preferred method to treat and thereby minimize or eliminate an existing, diagnosed or non-diagnosed, fungal or bacterial infection. Application of the invention as described herein and via the specific and novel mechanisms described herein will minimize and possibly eliminate manifestations of fungal and bacterial infections. Application of the invention as described herein will also minimize or possibly eliminate manifestations associated with the presence of pathogenic fungal and bacterial organisms, as identified above, in food or feed of mammalian and avian species.

### BRIEF DESCRIPTION OF THE DRAWINGS

The accompanying drawings and photographs which are incorporated into the following "Detailed Description of the Invention" form part of the specification and illustrate several aspects of the present invention and, together with the Detailed Description, serve to explain the details of the invention. In the following section five figures are presented.

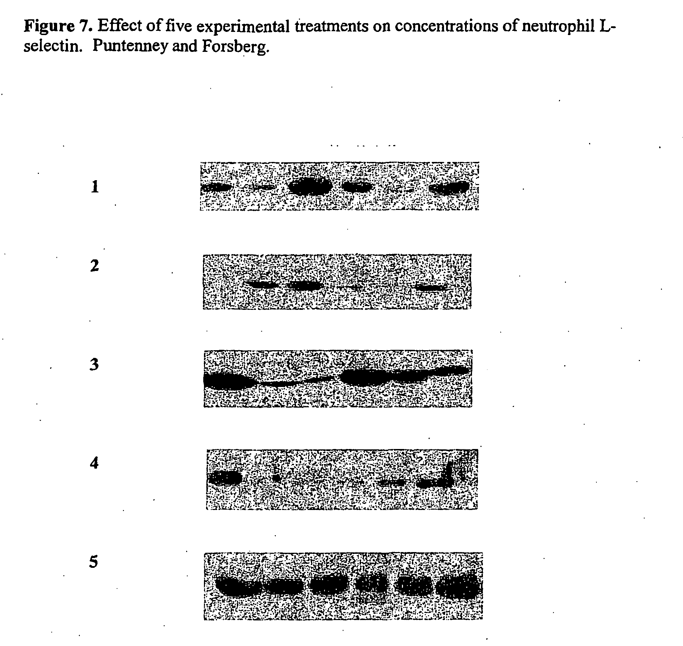

Figure 7. Effect of five experimental treatments on concentrations of neutrophil L-selectin. An experiment was conducted with 60 sheep. Twelve sheep were allocated to each treatment. The treatments consisted of:
1. Control
2. Immunosuppressed (daily injections of Azium [Dexamethasone], 0.1 mg/kg twice/day).
3. Immunosuppressed plus experimental product fed at 0.5%of daily dry matter intake.
4. Immunosuppressed plus moldy feed (addition of Aspergillus fumigatus-infected wheat mill run; 1.5 lbs/head/day).
5. Immunosuppressed plus moldy feed (as in Treatment 4) plus the experimental feed product as outlined in Treatment 3.

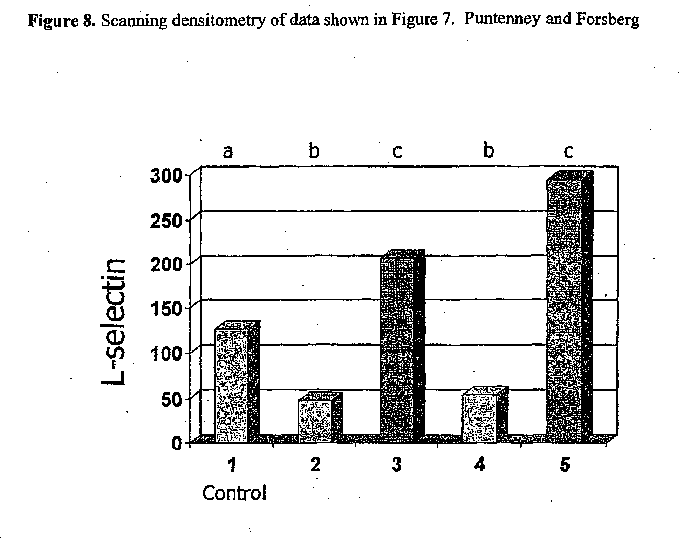

The duration of the trial was 28-days. On Day 28, blood was taken from six sheep per treatment and neutrophils were recovered by Percoll gradient centrifugation. The concentrations of L-selectin were determined by Western blot analysis using an antibody specific to L-selectin. Relative concentrations of L-selectin among the five treatment groups are shown in Figure 8.

**Figure 8****.** Scanning densitometry of data shown in Figure 7.

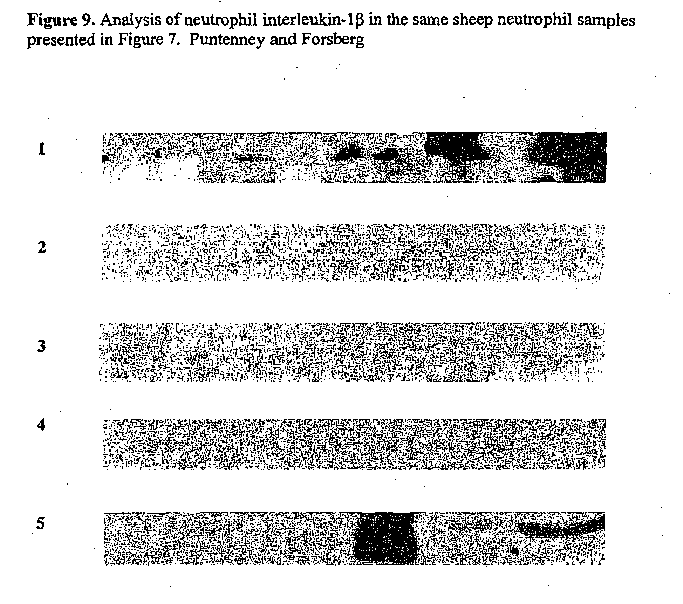

**Figure 9****.** Analysis of neutrophil interleukin-1β in the same sheep neutrophil samples presented in Figure 7.

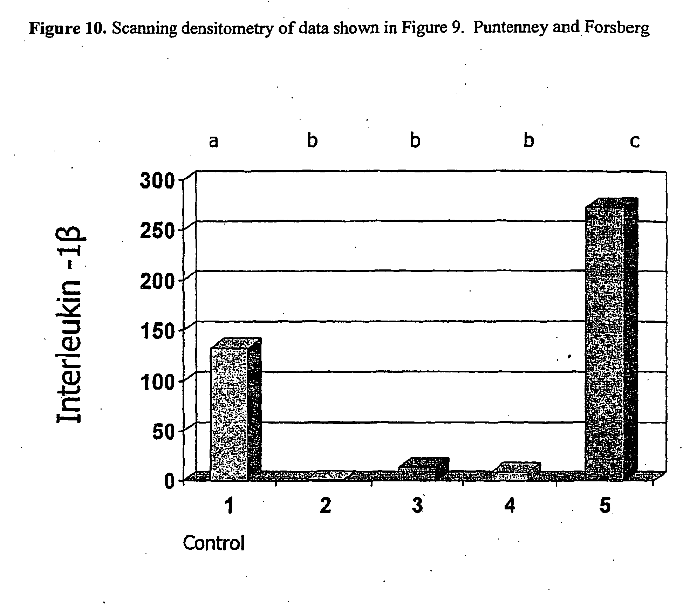

**Figure 10****.** Scanning densitometry of data shown in Figure 9.

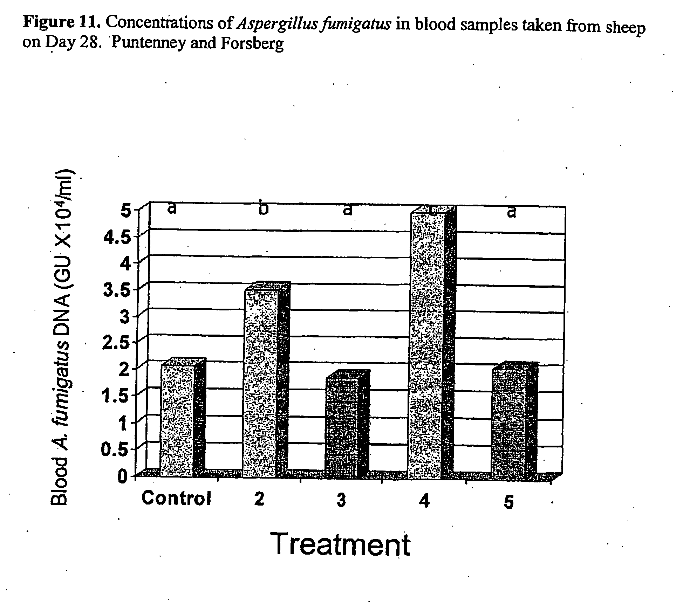

**Figure 11****.** Concentrations of *Aspergillus fumigatus* in blood samples taken from sheep on Day 28 of the above study. *A. fumigatus* DNA levels were assessed using a quantitative Sybr-Green PCR-based assay specific for *A. fumigatus.*

### DETAILED DESCRIPTION OF THE INVENTION

The present invention is based on the novel discovery that a combination of β-1,3 (4)-endoglucanohydrolase, β-1,3 (4)glucan, diatomaceous earth, mineral clay, and glucomannan effectively augments immune function and reduces colonization of tissues and blood by a pathogen.

The β-1,3 (4)-endoglucanohydrolase is from a commercial source and is produced from submerged fermentation of a strain of *Trichoderma longibrachiatum.*

The diatomaceous earth is prepared by methods commonly known in the art. It is available as a commercially-available acid-washed, product with 95% silica (SiO₂) and with its remaining components not assayed but consisting primarily of ash (minerals) as defined by the Association of Analytical Chemists (AOAC, 2002).

The yeast cell wall extract is prepared by a method commonly known in the art. It is a commercial source of β-1,3 (4)glucan and glucomannan derived from primary inactivated yeast *(Saccharomyces cerevisiae)* with the following chemical composition:

| | |
|---|---|
| Moisture | 2-3% |
| Dry matter | 97-98% |
| Proteins | 14-17% |
| Fats | 20-22% |
| Phosphorous | 1-2% |
| Mannans | 22-24% |
| β-1,3 (4)glucan | 24-26% |
| Ash | 3-5% |

The mineral clays (aluminosilicates) used in this invention may be fulfilled by any of a variety of commercially-available clays including, but not limited to, montmorillonite clay, bentonite and zeolite.

In a preferred embodiment of the invention, β-1,3 (4)-endoglucanohydrolase, diatomaceous earth, yeast cell wall extract and mineral clay are combined at 0.05-3%, 1-40%, 1-20% and 40-92%, respectively. In a preferred composition, β-1,3 (4)-endoglucanohydrolase, diatomaceous earth, yeast cell wall extract and mineral clay are combined at 0.1-3%, 5-40%, 2-10% and 40-80%, respectively. In an especially preferred embodiment of the invention, β-1,3 (4)-endoglucanohydrolase, diatomaceous earth, yeast cell wall extract and mineral clay are combined at 0.2-3%, 30-40%, 4-6% and 50-65%, respectively. The preferred physical form of the invention is a dry, free-flowing powder which is suitable for direct inclusion into a feed, food product or as a supplement to a total mixed ration or diet.

The compositions provided by the present invention may be incorporated directly into commercially-available feeds or food products or fed as supplements to commercially-available feeds or food products. The composition contained in the present invention may be fed to any mammalian or avian species. The methods of the invention comprise augmenting immune function in mammalian and avian species. When incorporated directly into feeds, the present invention may be added to feeds in amounts ranging from 0.1 to 5 kg per ton of feed. In an especially preferred composition, the invention may be added to feeds in amounts ranging from 1-2 kg per ton of feed.

The composition contained in the present invention may be added to animal feedstuffs or to foods in amounts ranging from 0.0125% to 2% by weight of feed. In a preferred embodiment, the composition is added to animal feedstuffs or to food in amounts from 0.0625% to 1% by weight of feed. In an especially preferred embodiment, the invention is added in amounts from 0.125% to 0.5% by weight of feed.

Alternatively, the composition contained in the present invention may be fed directly to mammalian or avian species as a supplement in amounts 0.016 grams/kg to 0.37 grams/kg of live body weight per day. In an especially preferred embodiment, the invention may be provided to mammalian and avian species in amounts of 0.10 grams/kg to 0.20 grams/kg of body weight per day. One of skill and art can appreciate that the amount of the invention fed can vary depending upon the animal species, size of the animal and type of the feedstuff to which the invention is added.

The novel methods of this invention comprise the ability of a combination of β-1,3 (4)-endoglucanohydrolase, diatomaceous earth, yeast cell wall extract and clay to augment immune function. The benefits resulting from the application of the invention to mammalian species include, but are not limited to, reduced death losses, reduced incidence of mycotic abortion, reduced incidence of jejunal hemorrhage syndrome (dead gut syndrome), reduced incidence of scouring (diarrhea), improved growth rate, improved efficiency of growth, improved milk production, improved efficiency of milk production and reduced somatic cell counts in milk products (dairy animals). The benefits from the application of the invention to avian species include, but are not limited to, reduced death losses, improved growth and egg production, improved fertility, and reduced incidence of enteric diseases.

The following are intended to be illustrative of the invention, and are not to be considered restrictive of the scope of the invention as otherwise described herein.

### Example 1

An experiment was conducted using 60 growing male and female sheep. Sheep were allocated to one of the five treatments (seven females and five males per treatment):
1. Control.
2. Immunosuppressed (daily injections of Azium [Dexamethasone], 0.1 mg/kg twice/day).
3. Immunosuppressed plus the invention fed at 0.5%of daily dry matter intake.
4. Immunosuppressed plus moldy feed (addition of *Aspergillus fumigatus-*infected wheat mill run; 1.5 lbs/head/day).
5. Immunosuppressed plus moldy feed (as in Treatment 4) plus the invention as outlined in Treatment 3.

Animals were fed a dairy-type diet for a period of 28 days. Immunosuppression was mediated in Treatments 2, 3, 4 and 5 by daily injection of Azium using a high dose (a model of extreme stress: Weber *et al.,* 2001). Sheep on Treatments 4 and 5 were challenged with a pathogenic mold by feeding wheat mill run which had been contaminated with a pathogenic mold *(Aspergillus fumigatus).* Sheep on Treatments 3 and 5 were supplemented with the invention at a rate of 0.5% of their daily dry matter intake. Following 28 days, blood samples were taken via jugular puncture and the neutrophil fractions were isolated using Percoll density gradient centrifugation. Following this, samples of neutrophil protein were processed using sodium dodecyl sulfate polyacrylamide gel electrophoresis and Western blotting using antibodies which are specific for L-selectin and interleukin-1β. Relative concentrations of L-selectin and interleukin-1β were assessed using scanning densitometry.

Figures 7 and 8 demonstrate effects of the five experimental treatments on neutrophil L-selectin. Injection with Azium caused a marked reduction (P<0.05) in L-selectin and provides evidence that Azium injection was, in fact, immunosuppressive. Addition of mold to the diets had no effect (P>0.05) on L-selectin concentrations. Of interest, addition of the invention to feed (Treatments 3 and 5) caused restoration (augmentation: P<0.05) of L-selectin.

**Interpretation:** The novel invention successfully restored (augmented) normal levels neutrophil L-selectin. Restoration of L-selectin on neutrophil surfaces will re-establish their ability to monitor the endothelial lining for pathogens.

Figure 9 and 10 demonstrate effects of the five experimental treatments on neutrophil interleukin-1β concentrations. Azium treatment caused a marked reduction (P<0.05) in neutrophil interleukin-1β concentration. This demonstrates that Azium was immunosuppressive. The novel invention had no effect (P>0.05) on neutrophil interleukin-1β in the absence of a pathogen challenge (i.e., Treatment 3 versus Treatment 2); however, the invention caused a marked increase (P<0.05) in neutrophil interleukin-1β in the presence of a pathogen challenge (i.e., Treatment 5 versus Treatment 4).

**Interpretation.** Interleukin-1β is an important pro-inflammatory cytokine which enables the neutrophil to fulfill its role as a phagocyte. Ability of the feed product to restore interleukin-1β in the presence of a pathogen (*A. fumigatus*) demonstrates that pathogens potential effects of the invention on immune function.

Figure II shows the effects of the five experimental treatments on blood concentrations of *A. fumigatus. A. fumigatus* concentrations were determined using a Sybr-Green real-time quantitative polymerase chain reaction (PCR) assay developed in our laboratory. The results demonstrate that the invention reduced (P<0.05) *A. fumigatus* concentration in blood.

**Interpretation.** The restoration of neutrophil function shown in Figures 7-10 manifests itself by reducing pathogen load detected within the blood compartment. The invention reduces pathogen load.

These results show that the composition of the invention (i.e., mineral clay, yeast cell wall extract, diatomaceous earth and β-1,3 (4)-endoglucanohydrolase) is capable of a previously-undescribed effect of augmenting immune function. The invention specifically restores levels of L-selectin and interleukin-1β in neutrophils thereby restoring the ability of neutrophils to monitor for the presence of invading pathogens.

The combination of products augments immunity in mammalian and domestic species and thereby prevents the invasion and colonization of the blood compartment. It represents a mixture which is flowable and easily incorporated into feed products and food products. The present invention was effective in achieving its immunostimulatory effects under growth conditions which might be found in mammalian and avian digestive systems where nutrients, moisture, oxygen and elevated temperatures are provided by the host.

The foregoing description of the preferred embodiment of the invention has been presented for purposes of illustration and description. It is not intended to be exhaustive or to limit the invention to the precise form disclosed. Obvious modifications or variations are possible in light of the above illustrations. The embodiment was chosen and described to provide the best illustration of the principles of the invention and its practical application to thereby enable one of ordinary skill in the art to utilize the invention in various embodiments and with modifications as are suited to the particular use contemplated. All such modifications and variations are within the scope of the invention as determined by the appended claims when interpreted in accordance with the breadth to which they are fairly, legally and equitably entitled.

**Table 1. Summary of mechanisms by which neutrophils can recognize/bind to pathogen prior to phagocytosis.**

| **Neutrophil receptor** | **PAMP¹ or Ligand** | **Comment** |
|---|---|---|
| **CD14** | lipopolysaccharide | direct binding to pathogen |
| **CD18** | lipopolysaccharide, | direct binding to pathogen |
| **TLR2** | lipoprotein, peptidoglycan, lipotechoic acid | direct binding to pathogen |
| **TLR3** | virus-derived double-stranded RNA | direct binding to pathogen |
| **TLR4** | lipopolysaccharide | direct binding to pathogen |
| **TLR5** | Flagellin | direct binding to pathogen |
| **TLR7/8** | Small synthetic anti-viral molecules | direct binding to pathogen |
| **TLR** 9 | unmethylated CpG DNA | direct binding to pathogen |
| **C3b/C3bi** | Complement factors | binds opsonized pathogen |
| **Fc** | "constant region" of antibodies | binds opsonized pathogen |

| | | |
|---|---|---|
| **1- PAMP: Pattern-associated molecular pattern** | | |

## Claims

1. A composition comprising a combination of β-glucan, glucomannan, β-1,3(4)-endoglucanohydrolase, calcined diatomaceous earth, and a mineral clay; for use in augmenting innate immune function in non-human animal species and thereby reducing susceptibility to an infection.

2. The composition of Claim 1, wherein the innate immune system is augmented in mammalian and avian species, preferably in immunosuppressed mammalian and avian species.

3. The composition of any of the Claims 1 or 2, wherein administering the composition results in change of indexes of innate immune function selected from the group consisting of an increase in neutrophil function, an increase in expression levels of neutrophil L-selectin, an increase in the expression levels of interteukin-1β, and a reduction in pathogen load and combinations thereof.

4. The composition of any of Claims 1-3, admixed in foods or foodstuffs of the non-human animal species.

5. The composition of Claim 1, wherein the combination of β-glucan, glucomannan, β-1,3(4)-endoglucanohydrolase, calcined diatomaceous earth, and a mineral clay, is for use in inhibiting fungal growth in digesta of the non-human animal species.

6. The composition of any of the Claims 2-5, wherein the mammalian species include all ruminant animals, preferably including dairy cattle, beef cattle or sheep, and/or the avian species include poultry species used in commercial livestock production.

7. The composition of any of Claims 1-6, wherein the mineral clay is montmorillonite, bentonite, aluminosilicate, zeolite clays, or mixtures thereof.

8. The composition of any of Claims 1-7, wherein the β-1,3(4)-endoglucanohydrolase is produced from submerged fermentation of *Trichoderma longibrachiatum.*

9. The composition of any of Claims 1-8, wherein the β-glucan and glucomannan are derived from boiling and enzyme autolysis of gram positive yeast cell walls from the genera of *Saccharomyces,* preferably the β-glucan and glucomannan are derived from boiling and enzyme autolysis of gram positive yeast cell walls from *Saccharomyces cerevisiae.*

10. The composition of any of Claims 1-9, wherein the diatomaceous earth is calcined at a minimum temperature of 900°C.

11. The composition of any of Claims 1-10, wherein the composition comprises between 15% and 40% diatomaceous earth, between 50% and 81% mineral clay, between 1.0% and 5.0% β-glucan, between 0.05% and 3.0% β-1,3(4)-endoglucanohydrolase and between 1% and 8% glucomannan.

12. The composition of any of Claims 1-11, wherein the composition comprises between 20% and 30% diatomaceous earth, between 60 and 75% mineral clay, between 1.0% and 3.5% β-glucan, between 0.1% and 3.0% β-1,3 (4)-endoglucanohydrolase and between 1.0% and 6.0% glucomannan.

13. A composition comprising a combination of β-glucan, glucomannan, β-1,3 (4)-endoglucanohydrolase, calcined diatomaceous earth, and a mineral clay for use in the augmentation of the immune function or for use in the treatment of pathogenic mold growth in a non-human animal selected from mammalian or avian species thereby augmenting the immune function to reduce susceptibility to an infection in the animal or thereby inhibiting the mold growth in the digesta passing through the animal to reduce susceptibility to a mycotic colonization of the digestive tract or to invastive mycoses.

14. The composition of claim 13, wherein the amount of the composition incorporated into the diet of the animal comprises between 0.0125% and 5% by weight of the animal's daily intake.

15. The composition of claim 13 or 14, wherein the infecting mold species include at least one of *Aspergillus, Aureobasidum, Candida, Eurotium, Fusarium, Mucor, Penicillium, Rachiborskiomyces* and other genera which are comprised in the fungal taxonomic classification.

16. The composition of any of Claims 13-15, wherein the mammalian species include all ruminant animals, preferably including dairy cattle, beef cattle or sheep, and/or the avian species include poultry species used in commercial livestock production.

17. The composition of any of Claims 13-16, wherein the mineral clay is montmorillonite, bentonite, aluminosilicate or zeolite clays, or mixtures thereof.

18. The composition of any of Claims 13-17, wherein the β-1,3(4)-endoglucanohydrolase is produced from submerged fermentation of *Trichoderma longibrachiatum.*

19. The composition of any of Claims 13-18, wherein the β-glucan and glucomannan are derived from boiling and enzyme autolysis of gram positive yeast cell walls from the genera of *Saccharomyces,* preferably the β-glucan and glucomannan are derived from boiling and enzyme autolysis of gram positive yeast cell walls from *Saccharomyces cerevisiae.*

20. The composition of any of Claims 13-19, wherein the diatomaceous earth is calcined at a minimum temperature of 900°C.

21. The composition of any of Claims 13-20, wherein the composition comprises between 15% and 40% diatomaceous earth, between 50% and 81% mineral clay, between 1.0% and 5.0% β-glucan, between 0.05% and 3.0% β-1,3(4)-endoglucanohydrolase and between 1% and 8% glucomannan.

22. The composition of any of Claims 13-21, wherein the composition comprises between 20% and 30% diatomaceous earth, between 60 and 75% mineral clay, between 1.0% and 3.5% β-glucan, between 0.1% and 3.0% β-1,3 (4)-endoglucanohydrolase and between 1.0% and 6.0% glucomannan.

23. Method for preparing a composition according to any of the claims 1-12 or 13-22.

24. Use of a composition comprising a combination of β-glucan, glucomannan, β-1,3(4)-endoglucanohydrolase, calcined diatomaceous earth, and a mineral clay in foods and animal feeds.

25. Foods and animal feeds comprising a combination of β-glucan, glucomannan, β-1,3(4)-endoglucanohydrolase, calcined diatomaceous earth, and a mineral clay.

## Patentansprüche

1. Zusammensetzung, umfassend eine Kombination aus β-Glucan, Glucomannan, β-1,3(4)-Endoglucanohydrolase, kalzinierter Kieselgur und einem Mineralton; zur Verwendung bei der Steigerung der angeborenen Immunfunktion in nicht humanen Lebewesenspezies und **dadurch** Verringerung der Anfälligkeit für eine Infektion.

2. Zusammensetzung nach Anspruch 1, wobei das angeborene Immunsystem in Säugetier- und Vogelspezies, vorzugsweise in immunsupprimierten Säugetier- und Vogelspezies, gesteigert wird.

3. Zusammensetzung nach Anspruch 1 oder 2, wobei das Verabreichen der Zusammensetzung resultiert in einer Veränderung von Indizes der angeborenen Immunfunktion, die ausgewählt sind aus jener Gruppe, die besteht aus einer Steigerung der neutrophilen Funktion, einer Steigerung der Expressionsniveaus von neutrophilem L-Selectin, einer Steigerung der Expressionsniveaus von Interleukin-1β und einer Verringerung der pathogenen Belastung sowie Kombinationen daraus.

4. Zusammensetzung nach einem der Ansprüche 1 - 3, die in Nahrungs- oder Futtermitteln der nicht humanen Lebewesenspezies beigemischt ist.

5. Zusammensetzung nach Anspruch 1, wobei die Kombination aus β-Glucan, Glucomannan, β-1,3(4)-Endoglucanohydrolase, kalzinierter Kieselgur und einem Mineralton dient zur Verwendung bei der Hemmung von Pilzwachstum in Digesta der nicht humanen Lebewesenspezies.

6. Zusammensetzung nach einem der Ansprüche 2 - 5, wobei die Säugetierspezies alle Wiederkäuer einschließen, wobei sie vorzugsweise Milchrind, Fleischrind oder Schaf einschließen, und/oder die Vogelspezies Geflügelspezies einschließen, die in kommerzieller Tierhaltung genutzt werden.

7. Zusammensetzung nach einem der Ansprüche 1 - 6, wobei der Mineralton Montmorillonit-, Bentonit-, Aluminosilicat-, Zeolithton oder Mischungen daraus darstellt.

8. Zusammensetzung nach einem der Ansprüche 1 - 7, wobei die β-1,3(4)-Endoglucanohydrolase aus der Submersfermentation von *Trichoderma longibrachiatum* hergestellt ist.

9. Zusammensetzung nach einem der Ansprüche 1 - 8, wobei das β-Glucan und das Glucomannan herrühren vom Kochen und von der Enzymautolyse grampositiver Hefezellwände von der Gattung *Saccharomyces,* vorzugsweise das β-Glucan und das Glucomannan herrühren vom Kochen und von der Enzymautolyse grampositiver Hefezellwände von *Saccharomyces cerevisiae.*

10. Zusammensetzung nach einem der Ansprüche 1 - 9, wobei die Kieselgur bei einer Mindesttemperatur von 900 °C kalziniert ist.

11. Zusammensetzung nach einem der Ansprüche 1 - 10, wobei die Zusammensetzung zwischen 15% und 40% Kieselgur, zwischen 50% und 81% Mineralton, zwischen 1,0% und 5,0% β-Glucan, zwischen 0,05% und 3,0% β-1,3(4)-Endoglucanohydrolase sowie zwischen 1% und 8% Glucomannan umfasst.

12. Zusammensetzung nach einem der Ansprüche 1 - 11, wobei die Zusammensetzung zwischen 20% und 30% Kieselgur, zwischen 60% und 75% Mineralton, zwischen 1,0% und 3,5% β-Glucan, zwischen 0,1% und 3,0% β-1,3(4)-Endoglucanohydrolase sowie zwischen 1,0% und 6,0% Glucomannan umfasst.

13. Zusammensetzung, umfassend eine Kombination aus β-Glucan, Glucomannan, β-1,3(4)-Endoglucanohydrolase, kalzinierter Kieselgur und einem Mineralton, zur Verwendung bei der Steigerung der Immunfunktion oder zur Verwendung bei der Behandlung pathogenen Schimmelwachstums in einem nicht humanen, aus Säugetier- oder Vogelspezies ausgewählten, Lebewesen, **dadurch** Steigern der Immunfunktion, um die Anfälligkeit für eine Infektion im Lebewesen zu verringern, oder **dadurch** Hemmen des Schimmelwachstums in den das Lebewesen passierenden Digesta, um die Anfälligkeit für eine mykotische Besiedelung des Verdauungstrakts oder für invasive Mykosen zu verringern.

14. Zusammensetzung nach Anspruch 13, wobei die Menge der Zusammensetzung, die in die Nahrung des Lebewesens einbezogen ist, zwischen 0,0125 Gew.-% und 5 Gew.-% der täglichen Aufnahme des Lebewesens umfasst.

15. Zusammensetzung nach Anspruch 13 oder 14, wobei die infizierenden Schimmelspezies zumindest eine Spezies von *Aspergillus, Aureobasidum, Candida, Eurotium, Fusarium, Mucor, Penicillium, Raciborskiomyces* und weiteren Gattungen einschließen, welche die pilztaxonomische Klassifikation umfasst.

16. Zusammensetzung nach einem der Ansprüche 13 - 15, wobei die Säugetierspezies alle Wiederkäuer einschließen, wobei sie vorzugsweise Milchrind, Fleischrind oder Schaf einschließen, und/oder die Vogelspezies Geflügelspezies einschließen, die in kommerzieller Tierhaltung genutzt werden.

17. Zusammensetzung nach einem der Ansprüche 13 - 16, wobei der Mineralton Montmorillonit-, Bentonit-, Aluminosilicat- oder Zeolithon oder Mischungen daraus darstellt.

18. Zusammensetzung nach einem der Ansprüche 13 - 17, wobei die β-1,3(4)-Endoglucanohydrolase aus der Submersfermentation von *Trichoderma longibrachiatum* hergestellt ist.

19. Zusammensetzung nach einem der Ansprüche 13 - 18, wobei das β-Glucan und das Glucomannan herrühren vom Kochen und von der Enzymautolyse grampositiver Hefezellwände von der Gattung *Saccharomyces,* vorzugsweise das β-Glucan und das Glucomannan herrühren vom Kochen und von der Enzymautolyse grampositiver Hefezellwände von *Saccharomyces cerevisiae.*

20. Zusammensetzung nach einem der Ansprüche 13 - 19, wobei die Kieselgur bei einer Mindesttemperatur von 900 °C kalziniert ist.

21. Zusammensetzung nach einem der Ansprüche 13 - 20, wobei die Zusammensetzung zwischen 15% und 40% Kieselgur, zwischen 50% und 81% Mineralton, zwischen 1,0% und 5,0% β-Glucan, zwischen 0,05% und 3,0% β-1,3(4)-Endoglucanohydrolase sowie zwischen 1% und 8% Glucomannan umfasst.

22. Zusammensetzung nach einem der Ansprüche 13 - 21, wobei die Zusammensetzung zwischen 20% und 30% Kieselgur, zwischen 60% und 75% Mineralton, zwischen 1,0% und 3,5% β-Glucan, zwischen 0,1% und 3,0% β-1,3(4)-Endoglucanohydrolase sowie zwischen 1,0% und 6,0% Glucomannan umfasst.

23. Verfahren zur Herstellung einer Zusammensetzung nach einem der Ansprüche 1 - 12 oder 13 - 22.

24. Verwendung einer Zusammensetzung, umfassend eine Kombination aus β-Glucan, Glucomannan, β-1,3(4)-Endoglucanohydrolase, kalzinierter Kieselgur und einem Mineralton in Lebensmitteln und Futtermitteln.

25. Lebensmittel und Futtermittel, umfassend eine Kombination aus β-Glucan, Glucomannan, β-1,3(4)-Endoglucanohydrolase, kalzinierter Kieselgur und einem Mineralton.

## Revendications

1. Composition comprenant un mélange de 5-glucane, de glucomannane, de β-1,3(4)-endoglucanohydrolase, de terre de diatomées calcinée et d'une argile minérale, destinée à améliorer la fonction immunitaire innée chez des espèces animales non humaines et à ainsi réduire la prédisposition à une infection.

2. Composition selon la revendication 1, dans laquelle le système immunitaire inné d'espèces mammifères et aviaires, de préférence d'espèces mammifères et aviaires immunodéprimées, subit une amélioration.

3. Composition selon l'une quelconque des revendications 1 ou 2, dans laquelle l'administration de la composition entraîne une modification des indicateurs de la fonction immunitaire innée choisis dans le groupe constitué par une augmentation de la fonction neutrophile, une augmentation des taux d'expression de la L-sélectine neutrophile, une augmentation des taux d'expression de l'interleukine-1β, et une réduction de la charge pathogène, ainsi qu'une combinaison de ceux-ci.

4. Composition selon l'une quelconque des revendications 1 à 4, mélangée à des denrées ou aliments destinés aux espèces animales non humaines.

5. Composition selon la revendication 1, dans laquelle le mélange de β-glucane, glucomannane, β-1,3(4)-endoglucanohydrolase, terre de diatomées calcinée et argile minérale est destiné à inhiber la croissance fongique dans le digesta des espèces animales non humaines.

6. Composition selon l'une quelconque des revendications 2 à 5, dans laquelle les espèces mammifères comprennent tous les animaux ruminants, y compris de préférence le bétail laitier, les bovins de boucherie ou les ovins, et/ou les espèces aviaires comprennent les espèces de volaille employées dans la production commerciale de viande.

7. Composition selon l'une quelconque des revendications 1 à 6, dans laquelle l'argile minérale consiste en de la montmorillonite, de la bentonite, de l'aluminosilicate, des argiles zéolitiques ou des mélanges de ceux-ci.

8. Composition selon l'une quelconque des revendications 1 à 7, dans laquelle la β-1,3(4)-endoglucanohydrolase est issue de la fermentation submergée de *Trichoderma longibrachiatum.*

9. Composition selon l'une quelconque des revendications 1 à 8, dans laquelle le β-glucane et le glucomannane sont produits par ébullition et autolyse enzymatique de parois cellulaires de levures gram-positives appartenant au genre *Saccharomyces,* le β-glucane et le glucomannane étant de préférence produits par ébullition et autolyse enzymatique de parois cellulaires de levures gram-positives *Saccharomyces cerevisiae.*

10. Composition selon l'une quelconque des revendications 1 à 9, dans laquelle la terre de diatomées a été calcinée à une température minimale de 900 °C.

11. Composition selon l'une quelconque des revendications 1 à 10, dans laquelle la composition comprend entre 15 % et 40 % de terre de diatomées, entre 50 % et 81 % d'argile minérale, entre 1,0 % et 5,0 % de β-glucane, entre 0,05 % et 3,0 % de β-1,3(4)-endoglucanohydrolase et entre 1 % et 8,0 % de glucomannane.

12. Composition selon l'une quelconque des revendications 1 à 11, dans laquelle la composition comprend entre 20 % et 30 % de terre de diatomées, entre 60 % et 75 % d'argile minérale, entre 1,0 % et 3,5 % de β-glucane, entre 0,1 % et 3,0 % de β-1,3(4)-endoglucanohydrolase et entre 1,0 % et 6,0 % de glucomannane.

13. Composition comprenant un mélange de β-glucane, de glucomannane, de β-1,3(4)-endoglucanohydrolase, de terre de diatomées calcinée et d'une argile minérale, destinée à augmenter la fonction immunitaire ou destinée à traiter la croissance de moisissures pathogènes chez un animal non humain choisi parmi des espèces mammifères ou aviaires en améliorant ainsi la fonction immunitaire pour réduire la prédisposition de l'animal à une infection ou en inhibant ainsi la croissance des moisissures dans le digesta de l'animal pour réduire la prédisposition à une colonisation mycosique du tube digestif ou à des mycoses invasives.

14. Procédé selon la revendication 13, dans laquelle la quantité de composition introduite dans le régime de l'animal est comprise entre 0,0125 % et 5 % en poids de l'apport journalier de l'animal.

15. Procédé selon la revendication 13 ou 14, dans laquelle les espèces de moisissures infectieuses comprennent au moins une espèce parmi *Aspergillus, Aureobasidium, Candida, Eurotium, Fusarium, Mucor, Penicillium, Rachiborskiomyces* et les autres genres de la classification taxonomique fongique.

16. Composition selon l'une quelconque des revendications 13 à 15, dans laquelle les espèces mammifères comprennent tous les animaux ruminants, y compris de préférence le bétail laitier, les bovins de boucherie ou les ovins, et/ou les espèces aviaires comprennent les espèces de volaille employées dans la production commerciale de viande.

17. Composition selon l'une quelconque des revendications 13 à 16, dans laquelle l'argile minérale consiste en de la montmorillonite, de la bentonite, de l'aluminosilicate, des argiles zéolitiques ou des mélanges de ceux-ci.

18. Composition selon l'une quelconque des revendications 13 à 17, dans laquelle la β-1,3(4)-endoglucanohydrolase est issue de la fermentation submergée de *Trichoderma longibrachiatum.*

19. Composition selon l'une quelconque des revendications 13 à 18, dans laquelle le β-glucane et le glucomannane sont produits par ébullition et autolyse enzymatique de parois cellulaires de levures gram-positives appartenant au genre *Saccharomyces,* le β-glucane et le glucomannane étant de préférence produits par ébullition et autolyse enzymatique de parois cellulaires de levures gram-positives *Saccharomyces cerevisiae.*

20. Composition selon l'une quelconque des revendications 13 à 19, dans laquelle la terre de diatomées a été calcinée à une température minimale de 900 °C.

21. Composition selon l'une quelconque des revendications 13 à 20, dans laquelle la composition comprend entre 15 % et 40 % de terre de diatomées, entre 50 % et 81 % d'argile minérale, entre 1,0 % et 5,0 % de β-glucane, entre 0,05 % et 3,0 % de β-1,3(4)-endoglucanohydrolase et entre 1 % et 8 % de glucomannane.

22. Composition selon l'une quelconque des revendications 13 à 21, dans laquelle la composition comprend entre 20 % et 30 % de terre de diatomées, entre 60 % et 75 % d'argile minérale, entre 1,0 % et 3,5 % de β-glucane, entre 0,1 % et 3,0 % de β-1,3(4)-endoglucanohydrolase et entre 1 % et 6 % de glucomannane.

23. Méthode pour la préparation d'une composition selon l'une quelconque des revendications 1 à 12 ou 13-22.

24. Utilisation d'une composition comprenant un mélange de β-glucane, de glucomannane, de β-1,3(4)-endoglucanohydrolase, de terre de diatomées calcinée et d'une argile minérale à des denrées et de nourriture animale.

25. Denrées et nourriture animale comprenant un mélange de β-glucane, de glucomannane, de β-1,3(4)-endoglucanohydrolase, de terre de diatomées calcinée et d'une argile minérale.
